# EUROPEAN PATENT APPLICATION

(11) **EP 3 444 355 A1**
(43) Date of publication of application: **20.02.2019**
(21) Application number: 17186457.2
(22) Date of filing: 16.08.2017
(51) Int. Cl.: C12P 17/04

(54) **PROCESS OF BIOCATALYTIC OXIDATION OF 5-(HYDROXYMETHYL)FURFURAL TO 2,5-DIFORMYLFURAN IN A TWO PHASE SYSTEM**

(71) Applicant: BASF SE, 67056 Ludwigshafen am Rhein (DE); University of Manchester, Manchester M13 9PL (GB); Danmarks Tekniske Universitet, 2800 Kgs. Lyngby (DK)
(72) Inventor: Breuer, Michael, 67056 Ludwigshafen (DE); Baldenius, Kai-Uwe, 67056 Ludwigshafen (DE); Birmingham, William, Manchester M13 9PL (GB); Pedersen, Asbjørn Toftgaard, 2800 Kgs. Lyngby (DK)
(74) Representative: BASF IP Association

(57) **Abstract**

Described is a process of biocatalytic oxidation of 5-(hydroxymethyl)furfural to 2,5-diformylfuran. The invention furthermore relates to the use of a specific compound as a constituent of a two phase system for biocatalytic oxidation of 5-(hydroxymethyl)furfural to 2,5-diformylfuran as well as to a two phase system for use in a process of biocatalytic oxidation of 5-(hydroxymethyl)furfural to 2,5-diformylfuran.

## Description

The present invention relates to a process of biocatalytic oxidation of 5-(hydroxymethyl)furfural to 2,5-diformylfuran. The invention furthermore relates to the use of a specific compound as a constituent of a two phase system for biocatalytic oxidation of 5-(hydroxymethyl)furfural to 2,5-diformylfuran as well as to a two phase system for use in a process of biocatalytic oxidation of 5-(hydroxymethyl)furfural to 2,5-diformylfuran.

The invention is defined in the attached claims. The present description discloses the invention and specific embodiments therof, as well as related aspects.

5-(hydroxymethyl)furfural (HMF; CAS: 67-47-0; 1) is a valuable aromatic platform chemical. 5-(hydroxymethyl)furfural is generally accessible from renewable raw materials, e.g. by dehydration of fructose. 5-(hydroxymethyl)furfural tends to decompose and polymerize, and develop brownish colour. It is thus preferred to convert 5-(hydroxymethyl)furfural to more stable compounds. 2,5-diformylfuran (DFF; CAS: 823-82-5; 2) is a more stable oxidation product of 5-(hydroxymethyl)furfural, which can be used as a starting material for polymerization or other follow-up-conversions. More specifically, 2,5-diformylfuran can be used as a building block and cross-linking agent in a range of different applications. For example, 2,5-diformylfuran can be used as a monomer for polymer production, e.g., in combination with urea, or can be further oxidized to useful building blocks such as formylfuran carboxylic acid (FFCA) and 2,5-furan dicarboxylic acid (FDCA).

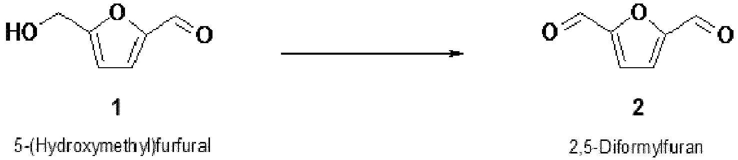

WO 2014/015256 discloses enzymatic methods for oxidation of 5-(hydroxymethyl)furfural and derivatives thereof, and in particular teaches enzymatic methods of oxidizing 5-(hydroxymethyl)furfural using a galactose oxidase and/or peroxygenase. Even more specifically, WO 2014/015256 A2 discloses a method of oxidizing 5-(hydroxymethyl)furfural, comprising contacting 5-(hydroxymethyl)furfural with a galactose oxidase in a reaction mixture under suitable conditions to provide 2,5-diformylfuran.

Galactose oxidase catalyzes the oxidation of primary alcohols to the respective aldehydes paralleled by the reduction of O₂ to H₂O₂, see Paukner, R. et al. PLoS ONE, 2014, 9, e100116/1. Generally, 5-(hydroxymethyl)furfural can be oxidized in the presence of an oxidoreductase as biocatalyst. Galactose oxidase is a preferred oxidoreductase due to its fairly broad substrate range. As galactose oxidase genes have been already heterologously expressed in numerous hosts, galactose oxidase is generally available for synthetic purposes. The enzyme code for galactose oxidase according to International Union of Biochemistry and Molecular Biology is EC 1.1.3.9.

Preferred oxidoreductases used for the oxidation of 5-(hydroxymethyl)furfural to 2,5-diformylfuran are generally those acting on the CH-OH-group of the donor (EC 1.1), and preferably those using molecular oxygen as terminal electron acceptor (EC 1.1.3)

The present invention in particular relates to processes of biocatalytic oxidation of 5-(hydroxymethyl)furfural to 2,5-diformylfuran in a reaction mixture comprising 5-(hydroxymethyl)furfural, water and an oxidoreductase as biocatalyst, preferably an oxidoreductase acting on the CH-OH-group of the donor (EC 1.1), more preferably an oxidoreductase using molecular oxygen as terminal electron acceptor (EC 1.1.3), and most preferably galactose oxidase (EC 1.1.3.9). Optionally the reaction mixture comprises one or more typical additives like buffer substances.

As known from own experiments, a significant problem with currently used processes of biocatalytic oxidation of 5-(hydroxymethyl)furfural to 2,5-diformylfuran is that the biocatalyst used is prone to deterioration by the starting material 5-(hydroxymethyl)furfural (and/or detrimental byproducts from 5-(hydroxymethyl)furfural synthesis) and, in particular, the reaction product 2,5-diformylfuran. This means that a biocatalyst is used which under the reaction conditions loses its biocatalytic properties or is even completely destroyed.

In view of the problems encountered as set forth above, it was a primary object of the present invention to provide for an improved process of biocatalytic oxidation of 5-(hydroxymethyl)furfural to 2,5-diformylfuran using an oxidoreductase as biocatalyst, wherein the problem caused by deterioration and/or inactivation of the biocatalyst is at least alleviated. More preferably, the biocatalyst should be protected from deterioration so that it can be used for an extended period of time in a continuous, semi-continuous or discontinuous process.

According to a first aspect of the present invention, the technical problem is solved by a process of biocatalytic oxidation of 5-(hydroxymethyl)furfural to 2,5-diformylfuran, comprising the following steps,
(a) providing or preparing a reaction system (in the form of a two or multi phase system, i.e, a reaction system having two, three or more phases), comprising
   (i) as a first phase (first liquid phase) a reaction mixture comprising
      5-(hydroxymethyl)furfural,
      an oxidoreductase as biocatalyst,
      water, and
      a buffer substance,
      and in contact with the first phase
   (ii) as a second phase (second liquid phase) a mixture comprising
      a solvent component and dissolved therein
      5-(hydroxymethyl)furfural,
   wherein the first and the second phase define an interface which allows for the transfer of compounds between the two phases,
(b) oxidizing 5-(hydroxymethyl)furfural to 2,5-diformylfuran by supplying oxygen to the reaction mixture of the first phase so that 5-(hydroxymethyl)furfural is oxidized to 2,5-diformylfuran in the presence of said oxidoreductase.

In the two phase system provided or prepared in step (a) of the process of the present invention, the biocatalyst is present in the aqueous first phase, but is virtually insoluble in the solvent component of the second (i.e. organic) phase. Thus, the solubility of the biocatalyst in water is higher than its solubility in the solvent component of the second phase. Preferably, the mass ratio of the amount of the biocatalyst in the second phase to the amount of the biocatalyst in the first phase is below 1:100, more preferably below 1:1000.

In the process of the present invention an oxidoreductase is used as biocatalyst. Oxidoreductase has the enzyme code EC 1 according to the conventions of the International Union of Biochemistry and Molecular Biology. Preferred oxidoreductases for use in the process of the present invention generally are those having enzyme code EC 1.1., i.e. are those acting on the CH-OH-group of the donor. More preferably, oxidoreductases with enzyme code EC 1.1.3 are used, i.e. those oxidoreductases using molecular oxygen as the terminal electron acceptor. Even more preferably, galactose oxidase having enzyme code EC 1.1.3.9 is used. Galactose oxidase is a copper dependent, monomeric enzyme. In nature, it is often found to be secreted by filamentous fungi. Galactose oxidase catalyses the oxidation of primary alcohols to the respective aldehydes paralleled by the reduction of O₂ to H₂O₂, see again Paukner, R. et al. PLoS ONE, 2014, 9, e100116/1. In document WO 2016/150629 it is disclosed how a galactose oxidase having enzyme code EC 1.1.3.9 can be prepared or provided. Furthermore, this document discloses a preferred enzyme of enzyme class 1.1.3.9. Biocatalysts of enzyme class EC 1.1.3.9 as disclosed in WO 2016/150629 are also preferred in the processes of the present invention.

The present invention is based on the surprising discovery that the biocatalyst used in a process of biocatalytic oxidation of 5-(hydroxymethyl)furfural to 2,5-diformylfuran maintains its activity for a longer time under process conditions if the process is conducted in the format of a two-phase-reaction (or multi phase reaction) with the selected solvents of this invention.

The buffer substances can be exogenous or endogenous which means that they are either deliberately added (exogenous) in order to adjust specific parameters of the aqueous mixture and/or allow for specific reactions (e. g. for the conversion of 5-(hydroxymethyl)furfural to 2,5-diformylfuran) or are present in the aqueous mixture simply because they were added or produced in earlier steps of an overall procedure (e. g., humic acids as typically prepared during 5-(hydroxymethyl)furfural synthesis have a buffering effect and therefore typically constitute endogenous buffer substances). Preferred are aqueous mixtures comprising a sufficient amount of endogenous and/or exogenous buffer substances buffering at pH 7.0 so that by addition of 0,5 mmol HCl per liter the pH does not drop below pH 6.5 and by addition of 0,5 mmol NaOH per liter the pH does not rise above 7.5.

The buffer substances used as a constituent of the reaction mixture of the first phase can be selected from a broad range of buffer substances known from the prior art as being acceptable in combination with biocatalysts. In particular, suitable buffer substances are those disclosed in WO 2014/015256 and/or are selected from the group consisting of alkali or alkaline earth metal hydrogen carbonate and/or carbonate, 1,4-piperazinediethanesulfonic acid (PIPES), 4-morpholinepropanesulfonic acid (MOPS), 4-(2-hydroxyethyl)-1-piperazineethane-sulfonic acid (HEPES), triethanolamine, tris(hydroxymethyl)aminomethane (TRIS), citrate, and phosphate. Potassium or sodium hydrogen phosphates are particularly preferred phosphate buffers for use as a constituent of the reaction mixture of the first phase, in particular because no handling of solid substances is required when a corresponding commercially available buffer solution is employed. By using a buffer substance, preferably a potassium and/or sodium hydrogen phosphate, the pH of the first phase is preferably adjusted to and/or buffered in a pH range of from 5 to 9, more preferably 6 to 8, most preferably 6.5 to 7.5. Preferably, the total concentration of the preferred buffer substances potassium hydrogen phosphate and sodium hydrogen phosphate in the first phase is in the range of from 10 to 200 mM, more preferably in the range of from 80 to 120 mM. In own experiments a concentration of 100 mM resulted in a particularly favorable process.

The reaction mixture of the first phase can comprise further additives. E.g., the presence of a protective agent (protectant) or helper enzyme for the oxidoreductase employed in the first phase is typically preferred. Thus, a process of the invention is preferred, wherein the reaction mixture of the first phase additionally comprises one or more protective agents or helper enzymes selected from the group consisting of horse radish peroxidase and catalase.

Said protective agent (protectant) or helper enzyme preferably acts by preventing or alleviating oxidative stress. A preferred protectant is horse radish peroxidase (HRP).

Furthermore, the presence of additives which react with and/or remove H₂O₂ produced during oxidation is typically preferred as they also reduce oxidative stress. Preferably, a catalase is used as an additive for removing and/or reacting with H₂O₂.

Preferred is a process according to the present invention, wherein the amount of the solvent component of the second phase is selected such that at the beginning of step (b) there is more 5-(hydroxymethyl)furfural present in the second (i.e. organic) phase than in the first (i.e. aqueous) phase.

A process of the invention (preferably a process defined above as being preferred) is preferred wherein the solvent component of the second phase
- under the reaction conditions of step (b) has a higher boiling point than water and/or (preferably "and")
- has a boiling point above 100 °C at 1013 hPa, preferably a boiling point in the range of from 100 to 200 °C at 1013 hPa.

The solvent component of the second phase preferably has a high boiling point (higher than water and/or above 100 °C at 1013 hPa), Typically in oxidation step (b) of the product of the present invention oxygen is supplied to the reaction mixture by bubbling gaseous oxygen containing gas through the first phase. In particular under such conditions, a second phase comprising a solvent component having a low boiling point would be evaporated easily. This is avoided by the preferred selection of the solvent component.

As stated above, also the product of the oxidation reaction, i.e. 2,5-diformylfuran (DFF), has a detrimental effect on the biocatalyst used in the process of the present invention. Correspondingly, a process of the present invention (preferably defined above as being preferred) is preferred, wherein the solvent component of the second phase is selected so that 2,5-diformylfuran has a higher solubility in the second phase present when reaction step (b) is started and/or terminated than in the first phase, and wherein preferably the amount of the solvent component of the second phase is selected such that when reaction step (b) is terminated there is more 2,5-diformylfuran present in the second phase than in the first phase.

When the reaction product 2,5-diformylfuran (DFF) has a higher solubility in the second (liquid) phase present when reaction step (b) is started and/or terminated than in the first (liquid) phase, it is automatically transferred from the first phase (where it is produced) into the second phase, and correspondingly it cannot inhibit and/or inactivate the biocatalyst present in the first phase.

The protection of the biocatalyst from the inhibitory effects of the reaction product 2,5-diformylfuran is particularly effective when at the end of reaction step (b) (when reaction step (b) is terminated) there is more 2,5-diformylfuran present in the second phase than in the first phase. Under these conditions, the process of inhibition and/or inactivation is at least significantly slowed down in comparison with a comparative process conducted in a single aqueous phase.

In some preferred processes, the second (liquid) phase is the lower phase of the (two or multi phase) reaction system. Correspondingly, in such a preferred process of the invention the second phase has a density which is higher than the density of the first (liquid) phase. Preferred solvents for such a preferred process are halogenated organic compounds.

In a particularly preferred process of the present invention, the second phase is the upper phase of the (two or multi phase) reaction system, in relation to the first phase. Correspondingly, in a particularly preferred process of the invention the second phase has a density which is lower than the density of the first phase. The density of the second phase essentially depends on the density of the solvent component used, in the usual manner. Preferred solvents for such a preferred process are defined below, in particular with reference to compounds selected from the group consisting of organic esters and carbonates.

A process of the invention is preferred (preferably a process as defined above as being preferred), wherein the reaction temperature in oxidation step (b) is in the range of from 10 to 50°C, preferably in the range of from 15 to 40°C, more preferably in the range of from 20 to 35°C. These reaction temperatures in own experiments allowed to arrive at a high yield of 2,5-diformylfuran, a high degree of conversion, and a high stability of the biocatalyst used.

Preferred is a process of the present invention (preferably a process defined above as being preferred), which is a continuous or semi-continuous process, wherein preferably 5-(hydroxymethyl)furfural is continuously added to the reaction mixture and/or
solvent component for the second phase is continuously added
and/or
2,5-diformylfuran is continuously removed from the (two or multi phase) reaction system, preferably by a continuous drain from the second phase.

Particularly preferred is a continuous or semi-continuous process of the present invention, wherein both 5-(hydroxymethyl)furfural and the solvent component for the second phase are continuously added to the (already existing) second phase, preferably in admixture with each other. Thus, preferably an admixture of 5-(hydroxymethyl)furfural and a solvent component for the second phase is continuously added to the second phase already existing.

In some embodiments, it is however preferred to continuously add 5-(hydroxymethyl)furfural to the reaction mixture of the first phase or to the second phase without the parallel addition of solvent component for the second phase.

Preferably, in the process of the present invention, the oxygen in oxidation step (b) is supplied as O₂ as a technically pure gas or in a gas mixture, preferably as O₂ in air
and/or (preferably "and")
is supplied to the first phase or the agitated reaction mixture (i.e. typically an agitated mixture of the first liquid phase and the second liquid phase, where typically the phase boundaries cannot be identified easily due to agitation) in a continuous manner.

By adding oxygen a gaseous phase may be formed in the reaction mixture. As stated above, in many cases it is preferred to bubble an O₂ containing gas mixture through the first phase or the agitated reaction mixture in a continuous manner. Alternatively, oxygen may be supplied in a dissolved form without forming a gas phase.

In a preferred process of the present invention, the 2,5-diformylfuran is separated from a mixture comprising some or all of the other constituents of the second phase by
- reducing the temperature of the mixture
   and/or
- removing at least a fraction of the solvent component from the mixture, wherein said removing of at least a fraction of the solvent component from the mixture is preferably accomplished by evaporation, more preferably by distillation,
so that 2,5-diformylfuran precipitates.

As the process of the present invention is conducted in a reaction system, the presence of the second phase allows for an easier work-up and, in particular, separation of the 2,5-diformylfuran from the other compounds involved than in a comparative process conducted in a single aqueous phase.

A process of the present invention is preferred (preferably a process defined above as being preferred), wherein the solvent component of the second phase comprises one or more compounds having a log P_{oct/water} above 0.5, preferably above 0.7, and preferably having a log P_{oct/water} in the range of from 0.5 to 2.5, more preferably in the range of from 0.7 to 2.3, most preferably in the range of from 0.8 to 2.0.

The term "log P_{oct/water}" is the logarithm of the partition coefficient between octanol and water at a temperature of 25 °C, calculated using Advanced Chemistry Development (ACD/Labs) Software V11.02 (© 1994-2017 ACD/Labs).

Preferably, the solvent component of the second phase comprises one or more compounds selected from the group consisting of organic esters and carbonates.

On the other hand, the use of compounds selected from the group consisting of aldehydes and ketones is less preferred as according to own experiments the degree of biocatalytic conversion of 5-(hydroxymethyl)furfural is lower when a second phase is present comprising aldehydes and/or ketones. Without wishing to be bound by any theory, it is presently assumed that aldehydes and ketones even if present mainly in the second phase have a negative effect on the biocatalyst present in the reaction mixture of the first phase.

Particularly preferred is a process of the present invention wherein the solvent component of the second phase comprises one or more compounds selected from the group consisting of diethyl carbonate and butyl acetate. These two compounds in own experiments resulted in processes with a particularly high degree of conversion. Both these compounds have a boiling point at atmospheric pressure above 100°C (diethyl carbonate, boiling point 127 °C; butyl acetate, boiling point 126 °C) and a log P_{oct/water} above 0.7, particularly in the preferred range of from 0.8 to 2.0. Log P_{oct/water} diethyl carbonate: 1.089±0.277; ; Log P butyl acetate: 1.804±0.205; Calculated using Advanced Chemistry Development (ACD/Labs) Software V11.02 (© 1994-2017 ACD/Labs. Log P_{oct/water}

Particularly preferred are also processes of the present invention wherein the solvent component of the second phase has a boiling point above 100 °C at 1013 hPa (preferably a boiling point in the range of from 100 to 200 °C at 1013 hPa) and comprises one or more compounds having a log P_{oct/water} above 0.5, preferably above 0.7, and preferably having a log P_{oct/water} in the range of from 0.5 to 2.5, more preferably in the range of from 0.7 to 2.3, most preferably in the range of from 0.8 to 2.0. As stated above, diethyl carbonate and butyl acetate are such compounds.

Preferably, in the process of the present invention, the second phase does not comprise any aldehydes or ketones other than 5-(hydroxymethyl)furfural, 2,5-diformylfuran and side products from the oxidation of 5-(hydroxymethyl)furfural, for the reasons stated above.

A process of the present invention allows for a high degree of conversion of the starting material 5-(hydroxymethyl)furfural. Preferably, in a process of the present invention, the reaction is allowed to proceed until at least 50 % of the total amount of 5-(hydroxymethyl)furfural employed is converted, preferably until at least 90 % of the total amount of 5-(hydroxymethyl)furfural employed is converted.

In order to reach a high degree of conversion and in order to protect the biocatalyst used, a process of the invention is typically conducted at a specified pH-value. A process of the invention is preferred, wherein the pH in the reaction mixture of the first phase at least in oxidation step (b) is in the range of from 6 to 8, preferably 7.0 to 7.5
and/or (preferably "and")
wherein the buffer substance buffers in the range of from 6 to 8, preferably 7.0 to 7.5.

Preferred buffer substances for use in a process of the present invention are stated above. The use of phosphates is particularly preferred. In own experiments, a high degree of conversion was reached when potassium phosphate and/or sodium phosphate were used. Correspondingly, these buffer substances are particularly preferred.

The invention also relates to the use of a compound selected from the group consisting of organic esters and carbonates,
- in which at 20 °C and 1013 hPa 2,5-diformylfuran has a higher solubility than in water,
- which has a boiling point above 100 °C at 1013 hPa (preferably a boiling point in the range of from 100 to 200 °C at 1013 hPa), and
- which preferably has a density below 1000 kg/m³ at 20 °C and 1013 hPa,
as a constituent of a two or multi (i.e., 3 or more) phase system for biocatalytic oxidation of 5-(hydroxymethyl)furfural to 2,5-diformylfuran, preferably for use in a process of the invention as defined above (preferably for use in a process defined above as being preferred). Particularly preferred is the use of a compound selected from the group consisting of organic esters and carbonates as defined above, wherein said compound has a log P_{oct/water} above 0.5, preferably above 0.7, and preferably having a log P_{oct/water} in the range of from 0.5 to 2.5, more preferably in the range of from 0.7 to 2.3, most preferably in the range of from 0.8 to 2.0.

Particularly preferred is the use of a compound selected from the group consisting of diethyl carbonate and butyl acetate as a constituent of a two phase system for biocatalytic oxidation of 5-(hydroxymethyl)furfural to 2,5-diformylfuran (preferably for use in a process of the present invention as defined above).

Preferably, the compound used according to this aspect of the invention is used in an amount of at least 50 % by weight of the organic phase of the two phase system.

The present invention also relates to a two or multi phase system for use in a process of biocatalytic oxidation of 5-(hydroxymethyl)furfural to 2,5-diformylfuran, preferably for use in a process of the present invention as defined in the claims or as defined above (preferably for use in a process defined above as being preferred), comprising
(i) as a first phase (first liquid phase) a reaction mixture comprising
   5-(hydroxymethyl)furfural,
   an oxidoreductase as biocatalyst,
   water, and
   a buffer substance
   and in contact with the first phase
(ii) as a second phase (second liquid phase) a mixture comprising
   a solvent component and dissolved therein
   5-(hydroxymethyl)furfural,
wherein the first and the second phase define an interface which allows for the transfer of compounds between the two phases.

Throughout the present text, features identified as being preferred in the context of a process of biocatalytic oxidation of the present invention are also preferred with respect to the use of a compound of the present invention and with respect to the reaction system of the present invention, and vice versa. If not indicated otherwise, preferred features of a process of the present invention are preferably combined with other preferred features of such process.

Hereinafter, the invention is further described with respect to examples:

### Examples 1 and 2: Biocatalytic oxidation of 5-(hydroxymethyl)furfural to 2,5-diformylfuran in a reaction system using diethyl carbonate (example 1) OR butyl acetate (example 2) as organic solvent components:

### Step 1: Preparation of a reaction system:

In a 4 L glass vessel, the following components were mixed by stirring:
800 ml 100 mM potassium phosphate buffer pH 7,
30.7 g (250 mM fin. conc.) HMF (5-(hydroxymethyl)furfural; > 80% purity; starting material was purified prior to use by dissolving in reaction buffer and mixing with 5 g activated carbon and 20 g poly-(1-phenylethylen) (tradename: crosspure F, by BASF) over night at 4°C; the mixture was filtered and used in the bioconversion)
1.107 µl (880 U/ml) catalase (Sigma) (protective agent - helper enzyme)
7 mg (1.7 U/ml) peroxidase (Sigma) (helper enzyme)
40 mg (0.05 mg/ml fin. conc.) galactose oxidase
640 ml diethyl carbonate (example 1) OR butyl acetate (example 2)

A reaction system resulted comprising
- a first (aqueous, lower) phase comprising 5-(hydroxymethyl)furfural, galactose oxidase (i.e. an oxidoreductase as biocatalyst), catalase, peroxidase, water, and potassium phosphate (buffer)
   and
- a second (organic, upper) phase comprising diethyl carbonate (example 1) OR butyl acetate (example 2) as solvent component and dissolved therein HMF (5-(hydroxymethyl)furfural). The two phases defined an interface allowing for the transfer of compounds between the two phases.

### Step 2: Oxidation of 5-(hydroxymethyl)furfural to 2,5-diformylfuran by supplying oxygen to the reaction mixture of the first phase so that 5-(hydroxymethyl)furfural is oxidized to 2,5-diformylfuran in the presence of galactose oxidase

Oxygen was supplied to the reaction mixture of the first phase as O₂ in air in a continuous manner for 24 hours at 25 °C, while stirring. 2,5-diformylfuran (DFF) was formed by oxidation of the 5-(hydroxymethyl)furfural.

After 24h hours the reaction was terminated.

### Step 3: Work-Up:

First and second phase were separated and the separated aqueous phase (first phase) was extracted with the same volume of organic solvent (diethyl carbonate (example 1) OR butyl acetate (example 2)). The combined solvent fractions (from the second phase and from the extraction) were concentrated to dryness (rotavap), i.e the solvent component was removed from the solvent fraction mixture so that 2,5-diformylfuran precipitated.

### Result:

Using diethyl carbonate (example 1) OR butyl acetate (example 2) as solvent component during the bioconversion the loss of solvent by stripping could be reduced dramatically while increasing the productivity of the overall system. 2,5-diformylfuran was accumulated up to 44 g*l⁻¹ in the second (organic) phase.

As a positive consequence of the low protein loading there is hardly any issue regarding phase separation at the end of the conversion in examples 1 and 2.

Most of the 2,5-diformylfuran was detected in the second (organic) phase in examples 1 and 2. Examples 1 and 2 show that both diethyl carbonate (example 1) and butyl acetate (example 2) are suitable as constituents of a two phase system for biocatalytic oxidation of 5-(hydroxymethyl)furfural to 2,5-diformylfuran, in a process of the invention.

With the additional extraction almost all 2,5-diformylfuran produced could be recovered in examples 1 and 2.

## Claims

1. Process of biocatalytic oxidation of 5-(hydroxymethyl)furfural to 2,5-diformylfuran, comprising the following steps,
(a) providing or preparing a reaction system, comprising
(i) as a first phase a reaction mixture comprising
5-(hydroxymethyl)furfural,
an oxidoreductase as biocatalyst,
water, and
a buffer substance,
and in contact with the first phase
(ii) as a second phase a mixture comprising
i. a solvent component and dissolved therein
ii. 5-(hydroxymethyl)furfural,
wherein the first and the second phase define an interface which allows for the transfer of compounds between the two phases,
(b) oxidizing 5-(hydroxymethyl)furfural to 2,5-diformylfuran by supplying oxygen to the reaction mixture of the first phase so that 5-(hydroxymethyl)furfural is oxidized to 2,5-diformylfuran in the presence of said oxidoreductase.

2. Process according to claim 1, wherein
the amount of the solvent component of the second phase is selected such that at the beginning of step (b) there is more 5-(hydroxymethyl)furfural present in the second phase than in the first phase.

3. Process according to any of the preceding claims, wherein the solvent component of the second phase
- under the reaction conditions of step (b) has a higher boiling point than water
and/or
- has a boiling point above 100 °C at 1013 hPa, preferably a boiling point in the range of from 100 to 200 °C at 1013 hPa.

4. Process according to any of the preceding claims, wherein the solvent component of the second phase is selected so that 2,5-diformylfuran has a higher solubility in the second phase present when reaction step (b) is started and/or terminated than in the first phase,
and wherein preferably the amount of the solvent component of the second phase is selected such that when reaction step (b) is terminated there is more 2,5-diformylfuran present in the second phase than in the first phase.

5. Process according to any of the preceding claims, wherein the second phase is the upper phase of the reaction system.

6. Process according to any of the preceding claims, wherein the reaction temperature in oxidation step (b) is in the range of from 10 to 50°C, preferably in the range of from 15 to 40°C, more preferably in the range of from 20 to 35°C.

7. Process according to any of the preceding claims, wherein the process is a continuous or semi-continuous process, wherein preferably
5-(hydroxymethyl)furfural is continuously added to the reaction mixture and/or
solvent component for the second phase is continuously added
and/or
2,5-diformylfuran is continuously removed from the reaction system, preferably by a continuous drain.

8. Process according to any of the preceding claims, wherein the oxygen in oxidation step (b)
is supplied as 02 as a technically pure gas or in a gas mixture, preferably as 02 in air and/or
is supplied to the first phase or the agitated reaction mixture in a continuous manner.

9. Process according to any of the preceding claims, wherein the 2,5-diformylfuran is separated from a mixture comprising some or all of the other constituents of the second phase by
- reducing the temperature of the mixture
and/or
- removing at least a fraction of the solvent component from the mixture, wherein said removing of at least a fraction of the solvent component from the mixture is preferably accomplished by evaporation, more preferably by distillation,
so that 2,5-diformylfuran precipitates.

10. Process according to any of the preceding claims, wherein the solvent component of the second phase comprises one or more compounds having a log Poct/water above 0.5, preferably above 0.7, and preferably having a log Poct/water in the range of from 0.5 to 2.5, more preferably in the range of from 0.7 to 2.3, most preferably in the range of from 0.8 to 2.0.

11. Process according to any of the preceding claims, wherein the solvent component of the second phase comprises one or more compounds selected from the group consisting of organic esters and carbonates,
wherein the solvent component of the second phase preferably comprises one or more compounds selected from the group consisting of diethyl carbonate and butyl acetate
and/or
wherein the second phase does not comprise any aldehydes or ketones other than 5-(hydroxymethyl)furfural, 2,5-diformylfuran and side products from the oxidation of 5-(hydroxymethyl)furfural.

12. Process according to any of the preceding claims, wherein the reaction is allowed to proceed until at least 50 % of the total amount of 5-(hydroxymethyl)furfural employed is converted, preferably until at least 90 % of the total amount of 5-(hydroxymethyl)furfural employed is converted.

13. Process according to any of the preceding claims, wherein
the pH in the reaction mixture of the first phase at least in oxidation step (b) is in the range of from 6 to 8, preferably 7.0 to 7.5
and/or
wherein the buffer substance buffers in the range of from 6 to 8, preferably 7.0 to 7.5 and/or
wherein the reaction mixture of the first phase additionally comprises one or more protective agents or helper enzymes selected from the group consisting of horse radish peroxidase and catalase.

14. Use of a compound
selected from the group consisting of organic esters and carbonates,
- in which at 20 °C and 1013 hPa 2,5-diformylfuran has a higher solubility than in water,
- which has a boiling point above 100 °C at 1013 hPa, and
- which preferably has a density below 1000 kg/m3 at 20 °C and 1013 hPa,
as a constituent of a two or multi phase system for biocatalytic oxidation of 5-(hydroxymethyl)furfural to 2,5-diformylfuran, preferably for use in a process according to any of the preceding claims.

15. Two or multi phase system for use in a process of biocatalytic oxidation of 5-(hydroxymethyl)furfural to 2,5-diformylfuran, preferably for use in a process according to any of claims 1 to 13, comprising
(i) as a first phase a reaction mixture comprising
i. 5-(hydroxymethyl)furfural,
ii. an oxidoreductase as biocatalyst,
iii. water, and
iv. a buffer substance
and in contact with the first phase
(ii) as a second phase a mixture comprising
v. a solvent component and dissolved therein
vi. 5-(hydroxymethyl)furfural,
wherein the first and the second phase define an interface which allows for the transfer of compounds between the two phases.
